(19) Europäisches Patentamt
European Patent Office
Office européen des brevets

(11) **EP 2 520 241 B1**

(12) **EUROPÄISCHE PATENTSCHRIFT**

(45) Veröffentlichungstag und Bekanntmachung des Hinweises auf die Patenterteilung:
**26.10.2016  Patentblatt 2016/43**

(51) Int Cl.:
***A61B 18/12*** *(2006.01)*

(21) Anmeldenummer: **11164643.6**

(22) Anmeldetag: **03.05.2011**

(54) **Einrichtung zur Gewebefusion oder Koagulation durch gewebewiderstandsabhängig spannungsgeregelte elektrische Einwirkung**

Device for tissue fusion or coagulation by means of tissue resistance-dependent voltage-controlled electric force

Dispositif de fusion ou de coagulation tissulaire par un effet électrique à tension régulée en fonction de la résistance tissulaire

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**07.11.2012  Patentblatt 2012/45**

(73) Patentinhaber: **ERBE Elektromedizin GmbH
72072 Tübingen (DE)**

(72) Erfinder: **Schall, Heiko
72622 Nürtingen (DE)**

(74) Vertreter: **Rüger, Barthelt & Abel
Patentanwälte
Webergasse 3
73728 Esslingen (DE)**

(56) Entgegenhaltungen:
**EP-A1- 1 862 137          EP-A1- 2 221 017
WO-A1-99/65406          WO-A2-2010/142438**

EP 2 520 241 B1

**Beschreibung**

Die Erfindung betrifft eine Vorrichtung zur Gewebefusion oder Koagulation mittels mindestens einer Elektrode, die einen Strom in das zu behandelnde Gewebe einleitet.

Die Gewebefusion oder Koagulation durch eine Elektrode, die lokal Strom in Gewebe und/oder Gewebeflüssigkeit einleitet, ist z.B. aus der EP 1 862 137 A1 bekannt.

Aus der WO 2010/142438 A2 ist ein Gerät zur Bereitstellung von HF-Energie bekannt, die einem chirurgischen Instrument zur Einwirkung auf ein biologisches Gewebe über Leitungen zugeführt wird. Zur Kompensation des Leitungswiderstands wird vorgeschlagen, einen negativen Innenwiderstand der speisenden Quelle festzulegen, um so den Spannungsabfall über den Leitungen zu kompensieren.

Die WO 99/65406 A1 schlägt ein Gerät zur Bereitstellung Heizstrom für ein Schneidwerkzeug vor, mit dem biologisches Gewebe geschnitten werden soll. Das Skalpell weist an seinen Flanken einen flächenhaften Heizwiderstand auf, der durch den Stromfluss erwärmt wird, um das Gewebe an den Schnittflächen zu koagulieren. Dabei darf das Skalpell nicht überhitzt werden. Dazu wird eine Strom- oder Spannungsquelle mit negativem Innenwiderstand vorgeschlagen, die den Wert des eingeprägten Stroms oder der eingeprägten Spannung automatisch so regelt, dass die mittlere Temperatur des Schneidwerkzeugs konstant gehalten wird.

Aus der EP 2 221 017 ist eine Einrichtung zur Gewebefusion mittels hochfrequenten Wechselstroms bekannt. Die Einrichtung weist eine elektrische Quelle mit einem Ausgang auf, an dem sie eine hochfrequente Wechselspannung liefert. Es ist mindestens eine Elektrode vorgesehen, die an den Ausgang angeschlossen und zur Bewirkung eines chirurgischen Effekts mit einem biologischen Gewebe in Wechselwirkung zu bringen ist. Außerdem ist mindestens eine Gegenelektrode vorgesehen, die an den Ausgang angeschlossen und mit dem biologischen Gewebe in elektrische Verbindung zu bringen ist. Weiter weist sie einen Impedanzsensor zur fortwährenden Bestimmung des sich zwischen der Elektrode und der Gegenelektrode einstellenden elektrischen Gewebewiderstands auf, wobei der Impedanzsensor ein den elektrischen Widerstand kennzeichnendes Impedanzsignal erzeugt.

Während der Stromeinleitung in das Gewebe entstehen in der Umgebung der Elektrode Effekte, die zu einer Änderung der Gewebeimpedanz führen. Zu Beginn der Einwirkung weist das Gewebe eine Anfangsimpedanz auf, die schon kurz nach Beginn der Stromeinleitung auf einen geringeren Wert absinkt, was als "Phase I" bezeichnet wird. Nach einiger Zeit steigt die Gewebeimpedanz wieder an, was als "Phase II" bezeichnet wird. Die Gewebeimpedanz erreicht in dieser Phase II normalerweise Werte, die deutlich über der Anfangsimpedanz liegen. Der Impedanzanstieg flacht sich dann ab und erreicht gegebenenfalls einen stabilen Endwert, was als "Phase III" bezeichnet wird.

Die zeitlichen Längen der Phasen I und II und die Steilheit des Impedanzabfalls und des Impedanzstiegs bestimmen die Qualität des erreichten chirurgischen Ergebnisses.

Das System gemäß der EP 1 862 137 A1 versucht deshalb, den zeitlichen Verlauf der Gewebeimpedanz mit einer SollKurve in Übereinstimmung zu bringen. Zu diesem Zweck vergleicht das System den auf eine geeignete Weise gemessenen Ist-Wert der Gewebeimpedanz fortwährend mit dem für den jeweiligen Zeitpunkt geltenden Soll-Wert. Wird eine Abweichung festgestellt, wird eine Gegenmaßnahme vorgenommen, wie beispielsweise eine Vergrößerung oder Verringerung der Energieeinleitung in das Gewebe. Der hier zugrunde liegende regelungstechnische Ansatz kann jedoch an Grenzen stoßen, wenn Regelabweichungen auftreten. Diese können zu einer irreversiblen Änderung der Gewebestruktur geführt haben, wie es bei der Behandlung biologischer Gewebe z.B. durch Denaturierung von Eiweißen charakteristisch ist.

Es wird deshalb nach einer robusten und verlässlichen Einrichtung zur Durchführung der Gewebefusion oder Koagulation insbesondere der Gefäßanastomose gesucht.

Diese Aufgabe wird gemäß Anspruch 1 gelöst.

Die Einrichtung beruht auf der Einleitung eines Stroms in ein biologisches Gewebe mittels einer Elektrode sowie der Ausleitung des Stroms mittels einer beruht. Zwischen beiden Elektroden bildet biologisches Gewebe einen elektrischen Gewebewiderstand. Im Verlauf der Stromeinleitung ergeben sich verschiedene Phasen I, II sowie III im zeitlichen Verlauf des Gewebewiderstands.

Die zur Speisung der Elektrode verwendete elektrische Quelle wird prozessentsprechend geführt. Als Führungsgröße wird in zumindest einer der Phasen, vorzugsweise in Phase II, der zwischen der Elektrode und der Gegenelektrode erfasste Gewebewiderstand genutzt. Vorzugsweise wird die Grö-3-ße des gemessenen Widerstands zumindest in der Phase II zur Regulierung der Spannung der Quelle herangezogen. Dabei kann eine Änderung der Spannung der Quelle schaltungstechnisch durch entsprechende Steuerung der Quelle z.B. anhand einer vorgegebenen Kennlinie erreicht werden. Die widerstandsabhängige Spannungssteuerung der Quelle erfolgt vorzugsweise während der Phase II. Es kann damit der Zeitverlauf der Gewebeimpedanz während der Phase II auf ein gewünschtes Verhalten festgelegt werden. Insbesondere kann erreicht werden, dass ein zu schnelles Austrocknen des Gewebes und somit eine unzuträgliche Verkürzung der Phase II durch zu hohen Energieeintrag verhindert wird. Mit anderen Worten, die Phase II kann in festgelegter Prozesszeit ausgeführt werden. Dies dient der Prozesssicherheit und der Sicherstellung einer guten Gewebefusion.

Es kann sichergestellt werden, dass die Ablaufsteuerung zum Durchführen der Phase II mit einem Timer zu einem gewünschten chirurgischen Ergebnis gleich bleibender Qualität führt. Insbesondere wird durch die Regelung der Spannung der Quelle in Abhängigkeit von dem gemessenen Gewebewiderstand während der Phase II vermieden, dass durch vorschnellen, zu starken Energieeintrag in dem Gewebe Effekte erzielt werden, die sich durch späteres Reduzieren des Energieeintrags nicht mehr rückgängig machen lassen. Die vorgeschlagene Regel- oder Steuerstrategie ist deshalb für die Regulierung des teilweise irreversibel ablaufenden und somit hochgradig nichtlinearen Prozesses in der Phase II besonders zweckmäßig.

Üblicherweise umfasst eine Einrichtung zur Durchführung des Verfahrens mindestens ein Gerät zur Bereitstellung elektrischer Leistung, wobei dieses Gerät eine elektrische Quelle umfasst. Weiter gehört zu der Einrichtung ein an das Gerät angeschlossenes Instrument mit mindestens einer Elektrode. Die Elektrode dient zur Einleitung des elektrischen Stroms in das Gewebe. Ein Steuermodul erfasst den Widerstand des Gewebes und steuert die Spannung der Quelle entsprechend. Schon in Phase I kann mit gewebewiderstandsabhängiger Spannung gearbeitet werden. Es kann aber auch z. B. mit einer festen Spannung oder auch einem zeitabhängigen Spannungsverlauf der Quelle gearbeitet werden.

Weiter vorzugsweise enthält eine solche Einrichtung eine Instrumentenerkennung. Mittels der Instrumentenerkennung können einzelne Parameter festgelegt werden, mit denen das Verfahren in Phase II arbeitet. Solche Parameter sind beispielsweise eine Leerlaufspannung $u_0$, ein Bezugsstrom $i_0$ und/oder ein Kennlinienexponent N. Die Kennlinie, nach der die von der Quelle abgegebene Spannung $u_a$ in Abhängigkeit von dem Gewebewiderstand $R_a$ festgelegt wird, kann nach der Beziehung:

$$u_a = u_0 - u_0 \left( \frac{R_a \cdot i_0}{u_0} \right)^N$$

festgelegt sein.

Zu der Instrumentenerkennung können Codestecker oder sonstige Speichermittel, wie beispielsweise ein an dem Instrument vorgesehener Speicher oder jegliche andere geeignete Mittel vorgesehen sein, die es ermöglichen, wenigstens einen der oben genannten Parameter dem Instrument entsprechend auszuwählen. Auf diese Weise können für verschiedene Instrumente jeweils gleiche Prozesszeiten T2 für die Phase II erreicht werden. Damit wird einerseits eine hohe Qualität der Gewebefusion der Gewebekoagulation sowie bei der Gefäßanastomose erreicht. Auch erhöht dies die Behandlungssicherheit, weil der Chirurg sich an eine einheitliche Einwirkungszeit des Instruments auf das Gewebe gewöhnt und einstellt.

Bei den im Vorstehenden genannten Instrumenten kann es sich um monopolare, vorzugsweise aber um bipolare Instrumente, wie beispielsweise Gefäßklemmen handeln, deren beide Klemmschenkel als Elektrode und Gegenelektrode ausgebildet sind. Solche Gefäßklemmen dienen dem dauerhaften Gefäßverschluss. Ein solches Gerät klemmt ein Gefäß ab und verschließt es durch Verkleben gegenüberliegender aufeinander gepresster Gewebewände. Außerdem kann ein solches Instrument ein Messer zum Durchtrennen des verschlossenen Gefäßes enthalten.

Monopolare, im Rahmen der Erfindung benutzte Instrumente können beispielsweise platten- oder kugelförmige, schleifenförmige oder anderweitig ausgebildete Elektroden aufweisen. Die Gegenelektrode nimmt hier an dem chirurgischen Effekt nicht teil. Sie wird beispielsweise als Neutralelektrode an dem Patienten befestigt.

Weitere Einzelheiten vorteilhafter Ausführungsformen der Erfindung ergeben sich aus Unteransprüchen, der Zeichnung oder der Beschreibung. Es zeigen:

Fig. 1 eine schematische Darstellung einer Einrichtung zur Gefäßanastomose,

Fig. 2 ein Blockschaltbild der Einrichtung nach Fig. 1 zur Veranschaulichung der Funktionsweise,

Fig. 3 ein Diagramm zur Verdeutlichung der Arbeitsweise der Spannungsführung bei der Einrichtung nach Figur 1 und 2,

Fig. 4 ein Diagramm zur Veranschaulichung des Verlaufs des Gewebewiderstands über der Zeit und

Fig. 5 ein Gefäß während eines Gefäßverschlusses in schematischer, ausschnittsweiser Längsschnittdarstellung.

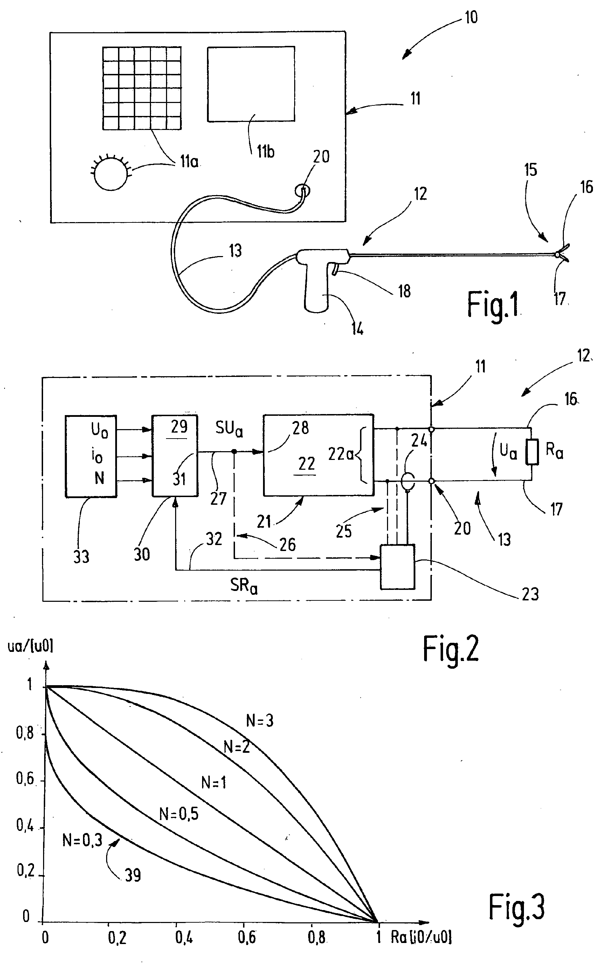

In Fig. 1 ist eine Einrichtung 10 veranschaulicht, die hier als Beispiel für unterschiedliche, zur Gewebekoagulation geeignete Einrichtungen steht. Die Einrichtung 10 umfasst ein Gerät 11 zur Speisung und zum Betrieb eines chirurgischen Instruments 12. Das Instrument 12 ist über eine Leitung 13 mit dem Gerät 11 verbunden. Das Instrument 12 wird über die Leitung 13 von dem Gerät 11 mit Spannung versorgt. Das Gerät 11 weist verschiedene Bedienelemente 11a und/oder ein oder mehrere Anzeigeelemente 11b, z.B. Displays auf.

Das Instrument 12 ist im vorliegenden Ausführungsbeispiel eine bipolare Gefäßklemme mit einem Handgriff 14 und einem Werkzeug 15. Letzteres umfasst eine Elektrode 16 und eine Gegenelektrode 17, von denen wenigstens eine, im vorliegenden Ausführungsbeispiel beide, beweglich gelagert sind. Sie können durch Betätigung eines Handhebels 18 aufeinander zu und voneinander weg bewegt werden. Weitere Elemente, z. B. Messer zum Durchtrennen von Gefäßen, Schalter oder dergleichen können vorgesehen sein.

Es wird darauf hingewiesen, dass es sich bei dem Instrument 12 nicht zwingend um ein bipolares Instrument handeln muss, wie es hier dargestellt ist. Es können auch monopolare Instrumente zum Einsatz kommen, die nur eine Elektrode aufweisen. Die Gegenelektrode ist dann beispielsweise eine am Patienten möglichst großflächig zu befestigende Neutralelektrode.

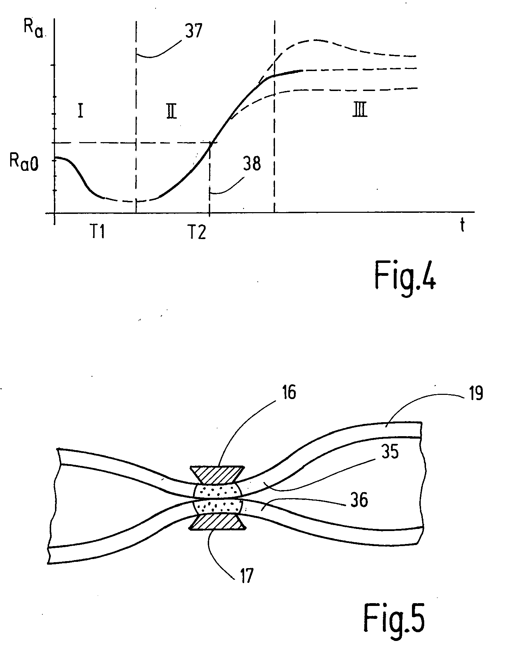

Die elektrische Grundstruktur der Einrichtung 10 ist in Fig. 2 als Blockschaltbild veranschaulicht. Ein Widerstand $R_a$ symbolisiert hier die Impedanz, im einfachsten Fall den Ohmschen Widerstand des zwischen den Elektroden 16, 17 gefassten Gewebes 19. Ein solches Gewebe 19 kann beispielsweise ein Blutgefäß sein, wie es in Fig. 5 ausschnittsweise und schematisch angedeutet ist. Die Elektroden 16, 17 sind in Fig. 2 lediglich auf ihre elektrische Funktion reduziert als Leitungen dargestellt. Sie sind über die Leitung 13 und einen Steckverbinder 20 an das Gerät 11 angeschlossen.

Zur Versorgung des Instruments 12 mit elektrischer Leistung dient eine Quelle 21, die durch eine Gleich- oder Wechselspannungsquelle, vorzugsweise durch einen HF-Generator 22 gebildet wird. Dieser stellt hochfrequente Wechselspannung, z.B. im Bereich mehrerer hundert Kilohertz und bedarfsweise mehreren hundert Volt, bei schneidenden, kontaktlosen Koagulationen oder abtragenden Anwendungen auch über tausend Volt bereit. Der HF-Generator 22 liefert die HF-Leistung an die Elektroden 16 und 17.

In einer der entsprechenden Leitungen, z.B. in der zu der Gegenelektrode 17 führenden Leitung, kann ein Impedanzsensor 23 angeordnet sein, der ein Signal erzeugt, das, je nach Ausführungsform, die komplexe Impedanz, den Betrag der Impedanz, den Blindanteil der Impedanz oder den ohmschen Gewebewiderstand $R_a$ des zwischen der Elektrode 16 und der Gegenelektrode 17 gefassten biologischen Gewebes 19 kennzeichnet.

Zur Erfassung der Gewebeimpedanz kann der Impedanzsensor 23 z.B. mit einem Stromfühler 24 verbunden sein, der an den Impedanzsensor 23 ein durch den Gewebewiderstand $R_a$ fließenden Strom kennzeichnet. Der Impedanzsensor kann daraus wegen der variierenden Ausgangsspannung $u_a$ nicht unmittelbar auf den Gewebewiderstand $R_a$ schließen. Im vorliegenden Ausführungsbeispiel erhält der Impedanzsensor 23 ein weiteres Signal, dass die Spannung $u_a$ kennzeichnet. Dieses Signal kann an den zu der Elektrode 16 und der Gegenelektrode 17 führenden Leitungen unmittelbar abgegriffen werden. Ein entsprechender Signalpfad 25 ist in Figur 2 gestrichelt eingetragen.

Alternativ kann, wenn die Quelle 21 einen ausreichend niedrigen Innenwiderstand hat ein der Ausgangsspannung $u_a$ entsprechendes Signal $su_a$ über einen alternativen Signalpfad 26 an den Impedanzsensor 23 herangeführt werden. Der Signalpfad 26 ist mit einem Signalpfad 27 verbunden, der das Signal $su_a$ als Steuersignal an einen Eingang 28 der Quelle 21, im vorliegenden Ausführungsbeispiel des HF-Generators 22, liefert. Das an dem Eingang 28 anliegende Signal $su_a$ legt die Größe der Ausgangsspannung $u_a$ an dem Ausgang 22a des HF-Generators 22 fest.

Das Gerät 11 enthält einen Kennlinienblock 29 mit einem Eingang 30 und einem Ausgang 31, von dem der Signalpfad 27 ausgeht. Der Kennlinienblock 29 setzt das über einen Signalpfad 32 von dem Impedanzsensor 23 erhaltene Impedanzsignal $SR_a$ in Ausgangsspannungssignal $su_a$ um. Der Kennlinienblock 29 realisiert folgende Gleichung:

$$su_a = u_0 - u_0 \left( \frac{SR_a \cdot i_0}{u_0} \right)^N$$

Wegen des vernachlässigbaren Innenwiderstands der Quelle 21 entspricht die Ausgangsspannung $u_a$ dem Ausgangsspannungssignal $su_a$. Außerdem entspricht die Gewebeimpedanz $R_a$ dem Gewebeimpedanzsignal $SR_a$. Es gilt deshalb:

$$u_a = u_0 - u_0 \left( \frac{R_a \cdot i_0}{u_0} \right)^N$$

Die von dem Kennlinienblock 29 festgelegen Kennlinien sind in Figur 3 veranschaulicht. Sie hängen von der Größe des Kennlinienexponenten N sowie außerdem von einem Bezugsstrom $i_0$ und der Leerlaufspannung $u_0$ ab. Diese Größen werden von einem Block 33 geliefert, der diese für den jeweiligen Betrieb konstanten Größen $u_0$, $i_0$ und N an den Kennlinienblock 29 übergibt.

Der Block 33 kann in einer einfachen Ausführungsform die drei Größen $u_0$, $i_0$ und N fest vorgeben. Es ist auch möglich, diese Größen durch die Bedienelemente 11a zumindest für sehr fachkundiges Personal zugänglich und einstellbar zu machen. In einer bevorzugten Ausführungsform enthält der Block 33 ein nicht weiter veranschaulichtes Instrumentenerkennungsmodul. Dieses kann bspw. mit einem an dem Instrument 12 vorgesehenen Speicher z.B. ein EPROM oder dergleichen kommunizieren. Dazu können nicht weiter veranschauliche Leitungsverbindungen oder auch die vorhandene Leitung 13 genutzt werden. Es kann außerdem eine RFID-Erkennung oder dergleichen vorgesehen sein. Alternativ kann der Stecker 20 als Codestecker ausgebildet sein, der die Bauart, den Typ, die Größe oder den Einsatzzweck des Instruments 12 kennzeichnet. Der Block 33 kann darauf eingerichtet sein, dies zu erkennen. Der Block 33 kann insoweit eine Zuordnung von verschiedenen erkannten Instrumenten oder Instrumententypen zu der jeweils passenden Leerlaufspannung $u_0$, dem passenden Bezugsstrom $i_0$ und/oder den passenden Kennlinienexponenten N vornehmen.

Der Impedanzsensor 23, der Kennlinienblock 29 und der Block 33 können durch spezifische Schaltungen oder durch Software realisiert werden. Sie können insbesondere Programme oder Programmabschnitte eines oder mehrerer Mikrocontroller sein.

Die insoweit beschriebene Einrichtung 10 arbeitet wie folgt:

Es wird davon ausgegangen, dass ein Operateur mit dem Instrument 12 ein Blutgefäß 19 verschließen möchte. Er fasst das Blutgefäß 19 deshalb zwischen der Elektrode 16 und der Gegenelektrode 17 und betätigt den Handhebel 18, um gegenüberliegende Abschnitte 35, 35 der Wand des Blutgefäßes 19 aneinander zu drücken, wie es Figur 5 zeigt. Eine geeignete Maßnahme, beispielsweise die Betätigung des Handhebels 18 oder auch die Betätigung eines weiteren Schalters, der z.B. an dem Handgriff 14 angebracht sein kann, aktiviert dann die Leistungsabgabe des Geräts 10.

Solange das Blutgefäß 19 noch nicht bestromt war, weist es eine Ausgangsimpedanz von z.B. 20 Ohm oder einen ähnlichen Wert auf. In Fig. 4 ist dies in dem Zeitintervall T1 links dargestellt. Auf der senkrechten Skala ist die Gewebeimpedanz $R_a$ logarithmisch veranschaulicht. Mit der Bestromung des Blutgefäßes 19 nimmt die Gewebeimpedanz $R_a$ relativ schnell ab z.B. öffnen sich einzelne Zellen und es bilden sich elektrolytgefüllte Strompfade. In dieser ersten Phase I kann nach einem geeigneten, fest vorgegebenen Schema gearbeitet werden, beispielsweise mit konstanter Spannung $u_a$, mit konstanter Leistung, mit konstantem Strom oder nach anderweitigen Kriterien. Dies kann fest vorgegeben sein. Es ist auch möglich, das Gerät 11 so zu gestalten, dass die einzelnen Modi für die Phase I z.B. durch die Bedienelemente wählbar oder einstellbar sind. Es ist auch möglich, eine Instrumentenerkennung vorzusehen, die beispielsweise mit einem in dem Instrument 12 vorgesehenen Speicher zusammenwirkt und dementsprechend die Betriebsart des HF-Generators 22 bzw. der Quelle 21 einstellt.

Während der Phase I wird der Fortgang des Prozesses überwacht, um den Beginn der Phase II zu erkennen. Die Überwachung kann beispielsweise durch eine Überwachung des fließenden Stroms oder durch Überwachung anderer physikalischer Größen, wie insbesondere auch des Phasenwinkels einer elektrischen Größe, des Stroms, der Spannung zwischen der Elektrode 16 und der Gegenelektrode 17 oder der Gewebeimpedanz $R_a$ erfolgen. Der Zeitverlauf derselben in Fig. 4 zeigt, dass es möglich ist, das erste Wiederansteigen des Gewebewiderstands $R_a$ nach Durchlaufen eines Minimums als Zeichen für den Beginn der Phase II zu nehmen. Dies ist in Fig. 4 durch eine vertikale gestrichelte Linie 37 veranschaulicht. Es ist jedoch auch möglich, andere Grenzwerte oder Schwellwerte zu setzen. Beispielsweise kann der Beginn der Phase II auch als derjenige Zeitpunkt definiert sein, bei dem erstmalig wieder

ein Gewebewiderstand $R_a$ erreicht wird, der deutlich über dem zu Beginn der Phase I gemessenen Gewebewiderstand $R_{a0}$ liegt. Dies ist in Fig. 4 durch eine zweite vertikale gestrichelte Linie 38 veranschaulicht.

Unabhängig davon, wie der Anfangszeitpunkt der Phase II definiert ist, geht das Gerät 11 zu Beginn der Phase II in eine Phase-II-Betriebsart über, die sich von der zuvor eingenommenen Betriebsart unterscheidet oder unterscheiden kann. In der Phase II führt das Gerät 11 die Ausgangsspannung $u_a$ entsprechend der von dem Kennlinienblock 29 vorgegebenen Kennlinie in Abhängigkeit von dem gemessenen Gewebewiderstand $R_a$. In Figur 3 ist von vielen möglichen Kennlinien eine Beispielkennlinie 39 markiert. Z.B. kann bei einem für diese Kennlinie geeigneten Instrument 11 die Ausgangsspannung $u_a$ entsprechend dem gemessenen Gewebewiderstand $R_a$ festgelegt werden. Wie ersichtlich weist das Gerät 11 aus Sicht des Instruments 12 einen (für $N \neq 0$) nichtlinearen negativen Innenwiderstand auf.

Der HF-Generator 22 selbst mag real einen geringen positiven Innenwiderstand oder einen Innenwiderstand von praktisch Null haben. Jedoch wird die Ausgangsspannung $u_a$ dem gemessenen Gewebewiderstand $R_a$ so nachgeführt, dass sich ein vorzugsweise nichtlinearer negativer Innenwiderstand der speisenden Quelle 21 ergibt. Vorzugsweise gilt dabei, dass der Betrag des so gebildeten negativen Innenwiderstands geringer ist als der Gewebewiderstand $R_a$.

Aufgrund der vorgegebenen Kennlinien folgt, dass ein abnehmender, durch das Gewebe fließender Strom $i_a$ zu einer abnehmenden Generatorspannung führt und umgekehrt. Auf diese Weise gilt, je schneller der Gewebewiderstand $R_a$ steigt, desto langsamer steigen die Spannung $u_a$ und somit der Energieeintrag in das Gewebe. Dies führt in der Phase II zu einer festgelegten Prozesszeit T2, siehe Fig. 4. Die Prozesszeit T2 kann somit für größere oder kleinere Gefäße und unabhängig von physiologischen Unterschieden zwischen einzelnen Patienten weitgehend konstant festgelegt werden und führt somit zu einer hohen Behandlungssicherheit und Behandlungsqualität.

Das Ende der Phase II ist erreicht, wenn die festgelegte Prozesszeit T2 abgelaufen ist. Der Koagulationsprozess kann dann in der Phase III noch nach geeigneten Vorgaben fortgesetzt oder auch beendet werden.

Bei dem zur Gewebefusion oder auch zur Koagulation geeigneten Verfahren wird nach Behandlungsbeginn des Gewebes, d.h. nach Durchlaufen einer Phase I, eine Phase II begonnen, innerhalb derer das biologische Gewebe für eine bestimmte festgelegte Prozesszeit mit moderatem Energieeintrag behandelt wird. Durch Festlegung eines funktionalen Zusammenhangs zwischen dem Gewebewiderstand $R_a$ und der Ausgangsspannung $u_a$ einer speisenden Quelle 21 kann erreicht werden, dass der Prozess in der Phase II eine Mindestbehandlungszeit nicht unterschreitet und somit eine vorzeitige Gewebeaustrocknung vermieden wird. Es wird eine ausreichende und verlässliche Verklebung der beteiligten Eiweiße im feuchten Milieu erreicht.

Bezugszeichenliste:

| 10 | Einrichtung |
|---|---|
| 11 | Gerät |
| 11a | Bedienelemente |
| 11b | Anzeigeelemente |
| 12 | Instrument |
| 13 | Leitung |
| 14 | Handgriff |
| 15 | Werkzeug |
| 16 | Elektrode |
| 17 | Gegenelektrode |
| 18 | Handhebel |
| 19 | Gefäß |
| 20 | Steckverbinder |
| 21 | Quelle |
| 22 | HF-Generator |
| 22a | Ausgang von 22 |
| 23 | Impedanzsensor |
| 24 | Stromfühler |
| 25 - 27 | Signalpfade |
| 28, 30 | Eingang |
| 29 | Kennlinienblock |
| 31 | Ausgang |
| 32 | Signalpfad |
| 33 | Block |
| 35, 36 | Wandabschnitte |
| 37, 38 | gestrichelte Linien - Beginn von Phase II |
| 39 | Beispielkennlinie |
| $N$ | Kennlinienexponent |
| $u_a$ | Ausgangsspannung |
| $u_0$ | Leerlaufspannung |
| $i_0$ | Bezugsstrom |
| $su_a$ | Ausgangspannungssignal |
| $SR_a$ | Impedanzsignal |
| $R_a$ | Gewebewiderstand |
| $R_{a0}$ | anfänglicher Gewebewiderstand |

**Patentansprüche**

1. Einrichtung zur Gewebefusion oder Koagulation mittels hochfrequenten Wechselstroms,
mit einer elektrischen Quelle (21), die einen Ausgang (22a) aufweist, an dem sie ausgeführt ist, zumindest zeitweilig mit einem negativen Innenwiderstand eine hochfrequente Wechselspannung zu liefern,
mit mindestens einer Elektrode (16), die an den Ausgang (22a) angeschlossen und zur Bewirkung eines chirurgischen Effekts mit einem biologischen Gewebe (19) in Wechselwirkung zu bringen ist,
mit mindestens einer Gegenelektrode (17), die an den Ausgang (22a) angeschlossen und mit dem biologischen Gewebe (19) in elektrische Verbindung zu bringen ist,
mit einem Impedanzsensor (23) zur fortwährenden Bestimmung des sich zwischen der Elektrode (16) und der Gegenelektrode (17) einstellenden elektrischen Gewebewiderstands ($R_a$), wobei der Impedanzsensor (23), ausgeführt ist, ein den elektrischen Widerstand kennzeichnendes Impedanzsignal ($SR_a$) zu erzeugen,
**dadurch gekennzeichnet, dass** die Einrichtung einen Kennlinienblock (29) aufweist, der mit dem Steuereingang (28) der Quelle (21) verbunden ist und dieser anhand des von dem Impedanzsensor (23) abgegebenen Impedanzsignals ($SR_a$) ein Ausgangsspannungssignal ($su_a$) bereitstellt, das an einem Eingang (28) der Quelle (21) liegt und die Größe von der elektrischen Quelle (21) abgegeben Spannung ($u_a$) festlegt,
wobei der Kennlinienblock (29) einen funktionalen Zusammenhang zwischen dem Ausgangsspannungssignal ($su_a$) und dem Impedanzsignal ($SR_a$) verkörpert und wobei der funktionale Zusammenhang Parameter enthält, wobei die Parameter eine Leerlaufspannung ($u_0$), ein Bezugsstrom ($i_0$) und ein Kennlinienexponent ($N$) sind, wobei der funktionale Zusammenhang durch die Beziehung:

$$u_a = u_0 - u_0 \left( \frac{R_a \cdot i_0}{u_0} \right)^N$$

gegeben ist, wobei der Kennlinienexponent eine positive oder negative, von Null verschiedene Zahl ist.

2. Einrichtung nach Anspruch 1, **dadurch gekennzeichnet, dass** wenigstens einer der Parameter durch eine Einrichtung zur Instrumentenerkennung festgelegt wird.

3. Einrichtung nach Anspruch 1, **dadurch gekennzeichnet, dass** die elektrische Quelle (21) eine hochfrequente Wechselspannungsquelle (22) ist.

4. Einrichtung nach Anspruch 1, **dadurch gekennzeichnet, dass** der Impedanzsensor (23) ausgeführt ist, den elektrischen Widerstand ($R_a$) im Rahmen seiner fortwährenden Bestimmung kontinuierlich zu messen.

5. Einrichtung nach Anspruch 1, **dadurch gekennzeichnet, dass** der Impedanzsensor (23) ausgeführt ist, den elektrischen Widerstand ($R_a$) im Rahmen seiner fortwährenden Bestimmung zeitdiskret zu messen.

**Claims**

1. Device for tissue fusion or coagulation by means of high-frequency alternating current, with an electrical source (21) having an output (22a) at which it is configured to supply, for at least part of the time, a high-frequency AC voltage with a negative internal resistance, with at least one electrode (16) which is connected to the output (22a) and to achieve a surgical effect is to be brought into interaction with a biological tissue (19), with at least one counter electrode (17) which is connected to the output (22a) and is to be brought into electrical connection with the biological tissue (19), with an impedance sensor (23) for continuous determination of the electrical tissue resistance ($R_a$) being set between the electrode (16) and the counter electrode (17), wherein the impedance sensor (23) is configured to generate an impedance signal ($SR_a$) characteristic of the electrical resistance, **characterised in that** the device has a characteristic curve block (29) which is connected to the control input (28) of the source (21) and which, using the impedance signal ($SR_a$) output by the impedance sensor (23), supplies said source with an output voltage signal ($su_a$) which lies at an input (28) of the source (21) and establishes the value of the voltage ($u_a$) output by the electrical source (21), wherein the characteristic curve block (29) embodies a functional correlation between the output voltage signal ($su_a$) and the impedance signal ($SR_a$), and wherein the functional correlation contains parameters, wherein the parameters are an idle voltage ($u_0$), a reference current ($i_0$) and a characteristic curve exponent (N), wherein the functional correlation is given by the equation:

$$u_a = u_0 - u_0 \left( \frac{R_0 \cdot i_0}{u_0} \right)^N$$

wherein the characteristic curve exponent is a positive or a negative figure different from zero.

2. Device according to claim 1, **characterised in that** at least one of the parameters is established by a device for instrument detection.

3. Device according to claim 1, **characterised in that** the electrical source (21) is a high-frequency AC voltage source (22).

4. Device according to claim 1, **characterised in that** the impedance sensor (23) is configured to measure the electrical resistance ($R_a$) continuously as part of a continuous determination.

5. Device according to claim 1, **characterised in that** the impedance sensor (23) is configured to measure the electrical resistance ($R_a$) at discrete times as part of a continuous determination.

**Revendications**

1. Dispositif de fusion tissulaire ou de coagulation au moyen de courant alternatif à haute fréquence, comportant une source électrique (21) dotée d'une sortie (22a), laquelle est configurée de manière à délivrer à ladite sortie, au moyen d'une résistance interne négative, au moins temporairement, une tension alternative à haute fréquence, comportant au moins une électrode (16) qui est raccordée à la sortie (22a) et qui doit être mise sous un effet alternatif en vue de provoquer un effet chirurgical sur un tissu biologique (19), comportant au moins une contre-électrode (17) qui est raccordée à la sortie (22a) et qui doit être mise en liaison électrique avec le tissu biologique (19), comportant un capteur d'impédance (23) destiné à déterminer en continu la résistance électrique tissulaire ($R_a$) qui doit être réglée entre l'électrode (16) et la contre-électrode (17), le capteur d'impédance (23) étant configuré de manière à produire un signal d'impédance ($SR_a$) qui caractérise la résistance électrique, **caractérisé en ce que** le dispositif présente un bloc (29) de courbes caractéristiques qui est relié à l'entrée de commande (28) de la source (21) et qui met à disposition de celle-ci, en relation avec le signal d'impédance ($SR_a$) délivré par le capteur d'impédance (23), un signal de tension de sortie ($su_a$) qui se situe à une entrée (28) de la source (21) et qui fixe la valeur de la tension ($u_a$) délivrée par la source électrique (21), le bloc (29) de courbes caractéristiques réalisant une liaison fonctionnelle entre le signal de tension de sortie ($su_a$) et le signal d'impédance ($SR_a$), et la liaison fonctionnelle comportant des paramètres, les paramètres étant une tension de fonctionnement à vide ($u_0$), un courant de référence ($i_0$) et un exposant (N) de caractéristiques, la liaison fonctionnelle étant obtenue par la relation :

$$u_a = u_0 - u_0 \left( \frac{R_a \cdot i_0}{u_0} \right)^N,$$

l'exposant de caractéristiques étant un nombre positif ou négatif, différent de zéro.

2. Dispositif selon la revendication 1 **caractérisé en ce qu'**au moins un des paramètres est fixé par un dispositif destiné à l'identification des instruments.

**3.** Dispositif selon la revendication 1 **caractérisé en ce que** la source électrique (21) est une source de tension alternative à haute fréquence (22).

**4.** Dispositif selon la revendication 1 **caractérisé en ce que** le capteur d'impédance (23) est configuré en vue de mesurer en continu la résistance électrique ($R_a$) dans le cadre de sa détermination continue.

**5.** Dispositif selon la revendication 1 **caractérisé en ce que** le capteur d'impédance (23) est configuré en vue de mesurer la résistance électrique ($R_a$) de façon discontinue dans le cadre de sa détermination continue.

Fig.1

Fig.2

Fig.3

Fig.4

Fig.5

**IN DER BESCHREIBUNG AUFGEFÜHRTE DOKUMENTE**

**In der Beschreibung aufgeführte Patentdokumente**

- EP 1862137 A1
- WO 2010142438 A2
- WO 9965406 A1
- EP 2221017 A